# EUROPEAN PATENT SPECIFICATION

(11) **EP 4 414 011 B1**
(45) Date of publication and mention of the grant of the patent: **13.08.2025**
(21) Application number: 24156788.2
(22) Date of filing: 09.02.2024
(51) Int. Cl.: A61M 16/01, A61M 16/10, A61M 16/18

(54) **SYSTEMS FOR MALIGNANT HYPERTHERMIA VAPORIZER USE PREVENTION**
SYSTEME ZUR VERHINDERUNG DER VERWENDUNG EINES BÖSARTIGEN HYPERTHERMIEVERDAMPFERS
SYSTÈMES POUR LA PRÉVENTION DE L'UTILISATION D'UN VAPORISATEUR D'HYPERTHERMIE MALIGNE

(30) Priority: 09.02.2023 US 202363444471 P
(43) Date of publication of application: 14.08.2024
(73) Proprietor: Dynasthetics, LLC, Salt Lake City, UT 84119 (US)
(72) Inventor: ORR, Joseph, Salt Lake City, 84119 (US); SAKATA, Derek, Salt Lake City, 84119 (US); BLACKWELL, Steven, Salt Lake City, 84119 (US)
(74) Representative: Wohlfahrt, Jan Günther

(56) References cited:
- US-B2- 8 485 187
- BILMEN J G ET AL: "The use of charcoal filters in malignant hyperthermia: have they found their place?", ANAESTHESIA, BLACKWELL SCIENCE LTD, GB, vol. 74, no. 1, 8 August 2018 (2018-08-08), pages 13 - 16, XP071009342, ISSN: 0003-2409, DOI: 10.1111/ANAE.14407
- CARLSON KEVIN J ET AL: "Management of patients susceptible to malignant hyperthermia: A surgeon's perspective", INTERNATIONAL JOURNAL OF PEDIATRIC OTORHINOLARYNGOLOGY, ELSEVIER, AMSTERDAM, NL, vol. 159, 30 May 2022 (2022-05-30), XP087115197, ISSN: 0165-5876, [retrieved on 20220530], DOI: 10.1016/J.IJPORL.2022.111187

## Description

### TECHNICAL FIELD

This disclosure relates generally to devices, systems, and methods for anesthesia vaporizer use prevention in patients having or susceptible to malignant hyperthermia.

### SUMMARY

The invention is defined by the appended claims. In various aspects, disclosed are systems and methods to prevent anesthetic vapors from reaching a patient that has, or is suspected of being susceptible to malignant hyperthermia. In some embodiments, a system to prevent anesthetic vapors from reaching a patient can include a pair of filters, such as activated charcoal filters, and a strap or connector disposed between or connecting the pair of filters. The strap or connector includes a release liner, a label disposed on a first section of the release liner, and at least one adhesive backed strip disposed on a second section of the release liner. The adhesive backed strip can include indicia (e.g., printings, markings, etc.) indicating that the patient may be susceptible to malignant hyperthermia. In some embodiments, the adhesive backed strip can be placed on controls or knobs of an anesthesia vaporizer after removal of the adhesive backed strip from the release liner to prevent inadvertent activation of the anesthesia vaporizer.

In some embodiments, a system to prevent anesthetic vapors from reaching a patient can include a pair of filters, where each filter of the pair is to be attached to an anesthesia vaporizer of an anesthesia machine. The filters can be activated charcoal filters or other filters, as appropriate. The system may additionally include a strap disposed between the pair of activated charcoal filters. The strap can include a release liner, a label attached to the release liner, and at least one adhesive backed-strip attached to the label. In some embodiments, the at least one adhesive-backed strip includes indicia indicating the patient may be susceptible to malignant hyperthermia. Additionally, the adhesive-backed strip can be placed on controls or knobs of the anesthesia vaporizer after removal from the label to prevent inadvertently turning on the anesthesia vaporizer.

Also disclosed are methods of preventing anesthetic vapors from reaching a patient. In some embodiments, the methods include providing a filter assembly and attaching one end of the filter assembly to an inspiratory limb and an expiratory limb of an anesthesia machine. The methods can also include attaching breathing hoses to an opposing end of the filter assembly, and capturing anesthetic vapors generated by the anesthesia machine before the anesthetic vapors reach the patient that is susceptible to or has malignant hyperthermia. In some embodiments, the filter assembly captures the anesthetic vapors. The methods also include preventing inadvertent activation of an anesthesia vaporizer of the anesthesia machine.

Other aspects of the disclosed subject matter, as well as features and advantages of various aspects of the disclosed subject matter, should be apparent to those of ordinary skill in the art through consideration of the ensuing description, the accompanying drawings, and the appended claims.

### BRIEF DESCRIPTION OF THE DRAWINGS

In the drawings:
FIG. 1 illustrates a plan view of one embodiment of a filter assembly, according to the present disclosure;
FIG. 2 illustrates a perspective view of the filter assembly of FIG. 1 installed or attached to an anesthesia machine;
FIG. 3 illustrates one embodiment of a strap used in a filter assembly, according to embodiments of the present disclosure;
FIG. 4 illustrates one embodiment of an adhesive backed strip (or tape) attached to anesthetic vaporizers of an anesthesia machine; and
FIG. 5 illustrates a flowchart of one embodiment of a method, not part of the invention.

### DETAILED DESCRIPTION

Most patients require exposure to anesthetic vapors to induce or cause amnesia and sedation during an operative procedure, such as a surgical procedure. However, some patients have a genetic disorder known as malignant hyperthermia. In these patients, even small amounts (e.g., >50 parts per million (ppm)) of anesthetic vapor can trigger the onset of uncontrollably high body temperatures and muscle rigidity, that can lead to death if untreated. When these patients require surgery, they are anesthetized using intravenous drugs rather than vapor anesthesia, but they can still require mechanical ventilation for breathing assistance during the procedure. Typically, mechanical ventilation during the procedure utilizes the same anesthesia machine used to administer anesthetic vapors. Breathing tubes (e.g., inspiratory and expiratory) are attached to the anesthesia machine to deliver oxygen and air (but not anesthetic vapors) to the patient.

Because administering anesthetic vapor directly to a malignant hyperthermia susceptible patient is potentially fatal, great care is taken by the anesthesia provider, physician, clinician, or other anesthesia provider, to prevent accidental anesthetic vapor administration from the anesthesia machine. Therefore, if a patient is suspected of being susceptible to (or having) malignant hyperthermia, some mechanism is required to capture or filter out the anesthetic vapors before they reach the malignant hyperthermic patient. For example, U.S. Patent Nos. 8,485,187 and 8,800,561 describe various systems, methods, and devices for removing volatile anesthetics from an anesthesia or ventilation system to minimize the effects of malignant hyperthermia in susceptible patients.

In some embodiments, activated charcoal filters of the present disclosure are placed on or connected to the anesthesia machine to capture and prevent residual anesthetic vapors from earlier patients who received an anesthetic using that same anesthesia machine from reaching the malignant hyperthermic patient. A strap or a connector, including a label, connects a pair of filters that are placed on the anesthesia machine. The label contains instructions for installation and use of the filters to prevent residual anesthetic vapors from reaching patients having or being susceptible to malignant hyperthermia.

For example, a system to prevent anesthetic vapors from reaching a patient can include a pair of filters, such as activated charcoal filters, and a strap or connector to connect the pair of filters. In some embodiments, the system includes a charcoal filter system that comprises a first charcoal filter, a second charcoal filter, and a connector that non-removably connects the first charcoal filter to the second charcoal filter. This non-removable connection can prevent the first charcoal filter from being removed from the second charcoal filter and used improperly, which could result in catastrophic consequences for patients with malignant hyperthermia. In other embodiments, the connector can be removed to allow separation of the first charcoal filter from the second charcoal filter.

In some embodiments, the system further includes one or more strips removably attached to the charcoal filter system. For example, the strip(s) can be attached to the strap or connector that connects the first charcoal filter to the second charcoal, or it can be attached to one or more of the filters. The strip can be, for example, a strip, a strap, a belt, a ring, a band, a tie, a binding, a buckle, etc. The strip can also be formed of any suitable material. In one embodiment, the strip can include an adhesive backing (e.g., an adhesive backed strip) and indicia (e.g., printings, markings, etc.) indicating that the patient may be susceptible to malignant hyperthermia. In one embodiment, the strap or connector that connects the filters can include a release liner, a label disposed on a first section of the release liner (e.g., a "peel-off" label), and at least one strip disposed on a second section of the release liner. In some embodiments, the adhesive backed strip can be placed on controls or knobs of an anesthesia vaporizer after removal of the adhesive backed strip from the release liner to prevent inadvertent activation of the anesthesia vaporizer.

In some embodiments, the strip can be an adhesive backed strip. Additionally, and/or alternatively, the strip can be removably attached to the connector for the charcoal filters, a strap of the charcoal filters, a release liner, a label, and/or to a filter (one or both) of the pair of filters. The strip can be removably attached via a perforated section, hook-and-loop fasteners, snaps, clips, magnets, adhesive, another appropriate removable attachment mechanism, or a combination thereof. The removable attachment of the strip allows the strip to be removed from the charcoal filter system (i.e., removed from the release liner of a strap between the filters, a label, and/or a filter), and be placed on controls (e.g., actuators, knobs, buttons, activators, etc.) of an anesthesia vaporizer. Placing the strip on controls of the anesthesia vaporizer prevents or reduces accidental activation of the controls and undesired anesthetic vapors from being delivered by the anesthesia vaporizer.

For example, placing the strip(s) on controls of the anesthesia vaporizer provides both a visual and tactile cue and/or reminder to the practitioner indicating that the patient may have or be susceptible to malignant hypothermia. Thus, a practitioner must either remove the strip(s) from the controls in the case of a non-malignant hypothermic patient or must install (or check the installation of) filters. Additionally, placing the strip(s) on controls of the anesthesia vaporizer prevents the controls from being actuated from incidental contact, such as a practitioner bumping into the controls. That is, the strip(s) may be secure enough to prevent accidental actuation of the controls due to incidental contact. Further, practitioners are often busy in preparation for and during a surgical procedure. Practitioners may be moving quickly and overlook whether or not a particular patient needs anesthesia vapor filters or whether such filters are in place. By placing the strip(s) on controls of the anesthesia vaporizer, a practitioner is forced to slow down and evaluate the settings of the procedure and ensure patient safety by installing the filters.

In some embodiments, a system to prevent anesthetic vapors from reaching a patient can include a pair of filters, where each filter of the pair is to be attached to an anesthesia vaporizer. The filters can be activated charcoal filters or other filters, as appropriate. In some embodiments, the system additionally includes a strap or connector disposed between the pair of activated charcoal filters. The strap can include a release liner, a label attached to the release liner, and at least one adhesive backed strip attached to the label. In some embodiments, the at least one adhesive backed strip includes indicia indicating the patient may be susceptible to malignant hyperthermia. Additionally, the at least one adhesive backed strip can be placed on controls or knobs of the anesthesia vaporizer after removal from the label to prevent inadvertently turning on the anesthesia vaporizer.

Also disclosed are methods of preventing anesthetic vapors from reaching a patient. In some embodiments, the methods include providing a filter assembly and attaching one end of the filter assembly to an inspiratory limb and an expiratory limb of an anesthesia machine. The methods can also include attaching breathing hoses to an opposing end of the filter assembly, and capturing anesthetic vapors generated by the anesthesia machine before the anesthetic vapor reaches the malignant hyperthermic patient. In some embodiments, the filter assembly captures the anesthetic vapors. In some embodiments, the methods also include preventing or reducing inadvertent activation of an anesthesia vaporizer of the anesthesia machine.

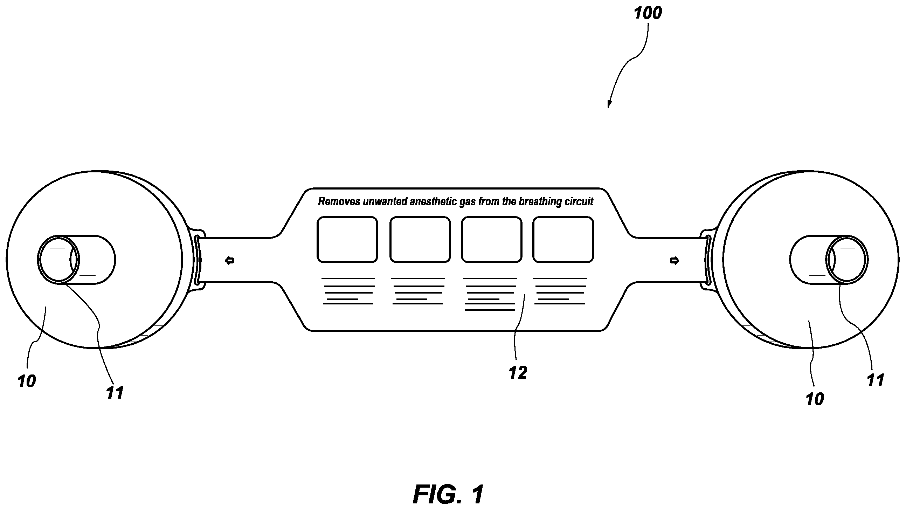

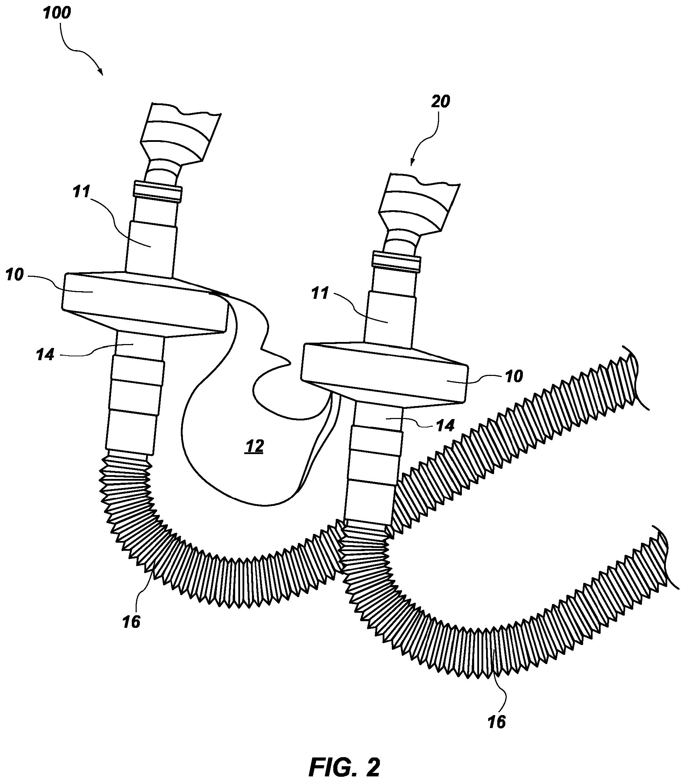

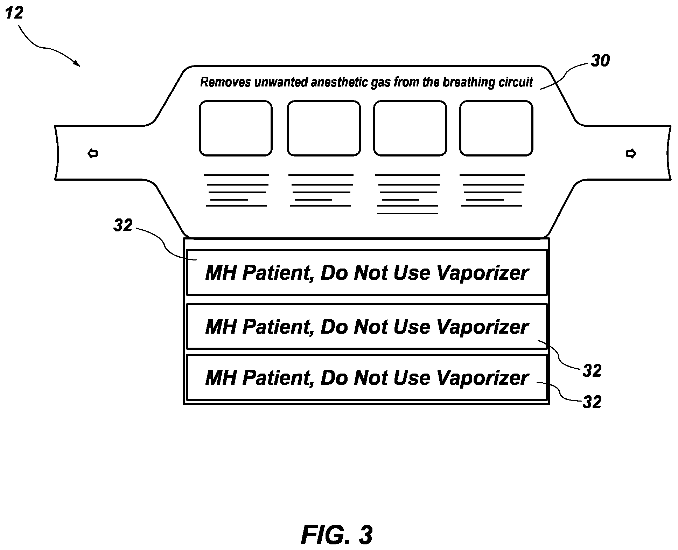

FIG. 1 illustrates a plan view of one embodiment of a filter assembly 100, according to the present disclosure. As illustrated, the filter assembly 100 includes a pair of filters 10 and a strap or connector 12 connecting the pair of filters 10. As discussed more fully below with respect to FIG. 3, the strap 12 can include instructions for installation and use of the filter assembly 100 with an anesthesia machine. Inclusion of the strap or connector 12 between the filters 10 ensures a clinician, physician, or other anesthesia provider, is aware of the medical condition(s) of the patient and properly installs the filters 10 in an anesthesia machine. Each filter 10 includes a first nozzle 11 for attaching the filter 10 to an anesthesia machine. For example, FIG. 2 illustrates a perspective view of the filter assembly 100 of FIG. 1 installed or attached to an anesthesia machine 20.

As illustrated, the first nozzle 11 of each filter 10 is attached to the anesthesia machine 20. Each filter 10 also includes a second nozzle 14, disposed on an opposing side of the filter 10 from the first nozzle 11. Breathing tubes or hoses 16 are attached to the second nozzle 14 of each filter 10 and facilitate mechanical ventilation for a patient undergoing, for example, a surgical procedure. Placing the filters 10 between the anesthesia machine 20 and the breathing tubes 16 allows the filters 10 to capture and filter out any residual anesthesia vapors contained within the anesthesia machine 20. This prevents the residual anesthesia vapors from traveling through the breathing tubes to the patient.

In some embodiments, the filters 10 can be activated charcoal filters. Any other type of suitable filter can also be used. In some embodiments, the filter 10 are capable of capturing and/or filtering out amounts anesthetic vapors ranging from 30 parts per million (ppm) to 150 ppm, such as 35, 40, 45, 50, 55, 60, 70, 80, 90, 100, 120, 130 ppm, or an amount of anesthetic vapors falling within a range defined by any two of the foregoing values. Capturing or filtering out these small amounts of anesthetic vapors prevents them from reaching the patient, who may be susceptible to malignant hyperthermia.

FIG. 3 illustrates one embodiment of a strap or connector 12 used in a filter assembly 100, according to the present disclosure. In some embodiments, the strap or connector 12 includes a label 30 and at least one strip 32 removably attached to the strap 12 and/or the label 30. For example, the at least one strip 32 can be an adhesive-backed strip 32, such that a backside of the strip contains adhesive to attach the strip to the label 30, a release liner (not illustrated), and/or a filter 10. In some embodiments, the strips 32 are attached to the label 30, release liner, and/or a filter 10 via a perforated section, adhesive backing, a hook-and-loop fastener, or a combination thereof. An adhesive-backed strip can refer to a strip 32 attached to the label 30, the release liner, and/or a filter 10 via one or more of a perforated section, adhesive backing, a hook-and-loop fastener, a combination thereof, or another removable attachment mechanism. Any type of attachment that allows the strip 32 to be removably attached to the filter assembly 100, and then removably attached to the anesthesia machine (as described below) can be used.

As illustrated, the strap or connector 12 includes three (3) adhesive backed strips 32, though in other embodiments more or fewer adhesive backed strips 32 can be included with the strap 12. In some embodiments, the number of adhesive-backed strips 32 attached to or included with the strap 12 corresponds to the number of controls for anesthesia vaporizers included in an anesthesia machine. In some embodiments, the label 30 and the at least one adhesive-backed strip 32 are attached or disposed on a release liner (not illustrated). The release liner can be a wax or silicon treated substrate (e.g., paper) that facilitates release of the label 30 and/or the at least one adhesive-backed strip 32 from the release liner. In some embodiments, the label 30 can be adhesive-backed to attach the label 30 to the release liner.

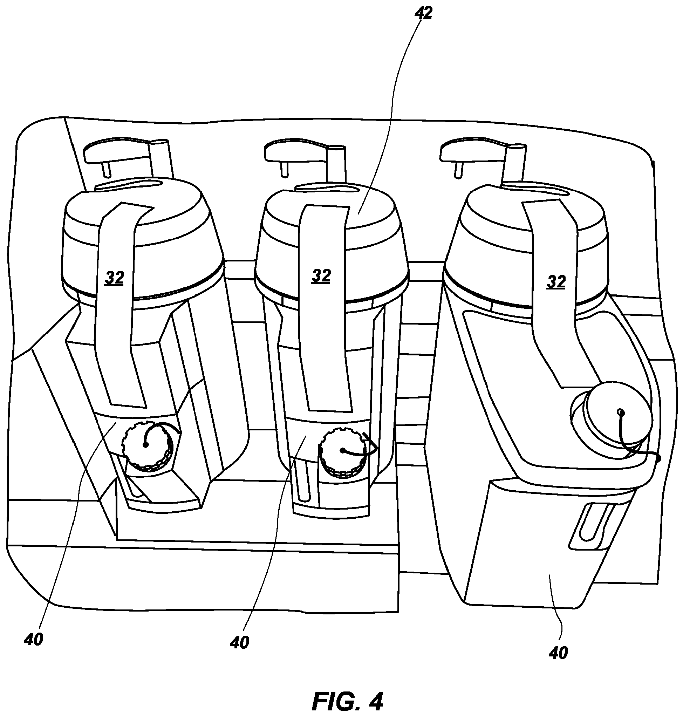

In some embodiments, the label 30 is attached to a first portion of the release liner and a second portion of the release liner receives the at least one adhesive-backed strip 32. In some embodiments, the label 30 is attached to the release liner and the at least one adhesive-backed strip 32 is attached to the label 30. For example, the at least one adhesive-backed strip 32 can be attached to the label 30 via one or more perforated sections. This allows the at least one adhesive-backed strip 32 to easily be removed from the label 30. This quick and easy release of the at least one adhesive-backed strip 32 increases the chances of successful installation of the at least one adhesive-backed strip 32 over controls of an anesthesia machine. As discussed more fully below with respect to FIG. 4, a removed adhesive-backed strip 32 can be placed on an anesthetic vaporizer to prevent incidental, inadvertent, or accidental activation of the anesthetic vaporizer.

In some embodiments, the label 30 includes indicia or markings providing instructions for installation and use of a filter assembly (e.g., filter assembly 100) with an anesthesia machine (e.g., anesthesia machine 20). In some embodiments, the at least one adhesive-backed strip 32 includes indicia indicating the patient has or may be susceptible to malignant hyperthermia and to not use an anesthetic vaporizer with that particular patient. This indicia can be a visual warning, such as a red label, a caution sign, etc. In some embodiments, the at least one adhesive-backed strip 32 includes indicia warning to remove the filters 10 at an end of a procedure for the patient. Other indicia can also be used as desired, such as labels for warnings regarding use of the muscle relaxant succinylcholine, and/or various anesthetic gases such as halothane, sevoflurane, desflurane, etc.

In other embodiments, there are no words or indicia on the adhesive-backed strip 32. In some embodiments, the at least one adhesive-backed strip 32 can be removed from the label 30 and/or the release liner, and placed on controls or knobs of an anesthetic vaporizer to prevent accidental or incidental activation of (e.g., turning on) the anesthetic vaporizer (see, e.g., FIG. 4). Where the strip 32 is not adhesive-backed, it can be attached to controls or knobs in any suitable manner such that the strip is physically covering at least a portion of the controls or knobs (such as folded over a portion of the controls or knobs, etc.).

In some embodiments, the release liner can include indicia, warnings, and/or instructions that become visible upon removal of one or more of the at least one adhesive-backed strips 32. For example, the release liner can include indicia advising removal of the filter assembly 100 (e.g., the pair of filters 10) from the anesthesia machine at an end of a procedure performed on the patient. As another nonlimiting example, the release liner can include indicia warning to discard already attached filters before using the anesthesia vaporizer.

FIG. 4 illustrates one embodiment of an adhesive-backed strip 32 attached to controllers 42 of anesthetic vaporizers 40 of an anesthesia machine 20. Specifically, the illustrated adhesive-backed strips 32 are placed over knobs or controls 40 of the anesthetic vaporizer to prevent accidental or incidental activation (e.g., turning "on") of the anesthetic vaporizers.

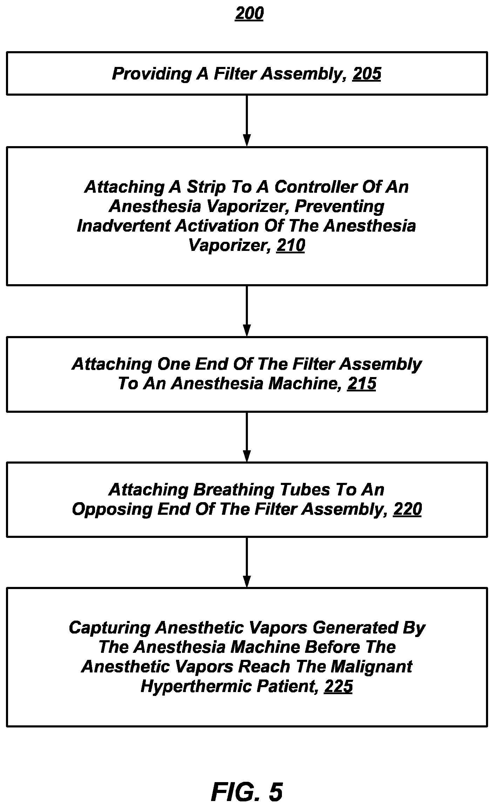

FIG. 5 illustrates a flowchart of one embodiment of a method 200, according to the present disclosure. In some embodiments, the method 200 can include providing a filter assembly, at 205. The filter assembly may include at least one removable strip. The filter assembly can be the filter assembly 100 of FIGS. 1 and 2, including a pair of filters connected by a strap, where the strap includes a label and one or more strips. The one or more strips can be attached to the label via a perforated section, adhesive backing, a hook-and-loop fastener, or a combination thereof. Alternatively, the one or more strips are removably attached to one or both of the filters in the pair of filters. The one or more strips may be removably attached to the filters (e.g., one or both) via a perforated section, adhesive backing, a hook-and-loop fastener, or a combination thereof.

The method 200 can include attaching a removable strip to a controller of an anesthesia vaporizer, preventing inadvertent activation of the anesthesia vaporizer, at 210. In some embodiments, one filter is attached to an inspiratory limb of the anesthesia machine and the other filter is attached to an expiratory limb of the anesthesia machine.

The method 200 can also include attaching one end of the filter assembly to an anesthesia machine, at 215, and attaching breathing hoses to an opposing end of the filter assembly, at 220. The method 200 can further include capturing anesthetic vapors generated by the anesthesia machine before the anesthetic vapors reach the malignant hyperthermic patient, at 225. In some embodiments, the filter assembly captures and filters out the anesthetic vapors. While the flowchart of FIG. 5 illustrates discrete acts show in a particular order, the acts could be performed in a different order, some acts could be performed simultaneously by one or more clinicians, etc. For example, the filter assembly can be attached before the removable strip is attached to a controller of the anesthesia vaporizer, at the same time, etc.

In one embodiment, the system includes one, two, three, four, or more removable strips. For example, the number of removable strips can correlate to the number of controls on an anesthesia vaporizer. Some anesthesia machines include one control, two controls, or three or more controls. The number of removable strips can correlate to the number of controls on the anesthesia machine so that each control can be covered, labeled, etc., to prevent or reduce accidental or inadvertent activation of the control for a patient that has, or is suspected to have, malignant hyperthermia.

While particular embodiments have been illustrated and described herein, it should be understood that various other changes and modifications may be made without departing from the scope of the claimed subject matter. Moreover, although various aspects of the claimed subject matter have been described herein, such aspects need not be utilized in combination.

In one embodiment, the terms "about" and "approximately" refer to numerical parameters within 10% of the indicated range. The terms "a," "an," "the," and similar referents used in the context of describing the embodiments of the present disclosure (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. All methods described herein can be performed in any suitable order unless otherwise indicated herein or otherwise clearly contradicted by context. The use of any and all examples, or exemplary language (e.g., "such as") provided herein is intended merely to better illuminate the embodiments of the present disclosure and does not pose a limitation on the scope of the present disclosure. No language in the specification should be construed as indicating any non-claimed element essential to the practice of the embodiments of the present disclosure.

Groupings of alternative elements or embodiments disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain embodiments are described herein, including the best mode known to the author(s) of this disclosure for carrying out the embodiments disclosed herein. Of course, variations on these described embodiments will become apparent to those of ordinary skill in the art upon reading the foregoing description. The author(s) expects skilled artisans to employ such variations as appropriate, and the author(s) intends for the embodiments of the present disclosure to be practiced otherwise than specifically described herein. Accordingly, this disclosure includes all modifications and equivalents of the subject matter recited in the claims appended hereto as permitted by applicable law. Moreover, any combination of the above-described elements in all possible variations thereof is encompassed by the present disclosure unless otherwise indicated herein or otherwise clearly contradicted by context.

Specific embodiments disclosed herein may be further limited in the claims using consisting of or consisting essentially of language. When used in the claims, whether as filed or added per amendment, the transition term "consisting of" excludes any element, step, or ingredient not specified in the claims. The transition term "consisting essentially of" limits the scope of a claim to the specified materials or steps and those that do not materially affect the basic and novel characteristic(s). Embodiments of this disclosure so claimed are inherently or expressly described and enabled herein.

Although this disclosure provides many specifics, these should not be construed as limiting the scope of any of the claims that follow, but merely as providing illustrations of some embodiments of elements and features of the disclosed subject matter. Other embodiments of the disclosed subject matter, and of their elements and features, may be devised which do not depart from the scope of any of the claims. Features from different embodiments may be employed in combination. Accordingly, the scope of each claim is limited only by its plain language and the legal equivalents thereto.

## Claims

1. A system (100) to prevent anesthetic vapors from reaching a patient, the system comprising an activated charcoal filter system comprising a first filter (10) and a second filter (10), a connector (12) connecting the first filter (10) to the second filter (10), wherein each of the first and second filters (10) is to be attached to an anesthesia vaporizer (40), **characterized in that** the system further comprises:
a strip (32) removably attached to at least a portion of the activated charcoal filter system (100), the strip (32) for removal from the activated charcoal filter system (100) and placement on a controller (42) of the anesthesia vaporizer (40) to prevent inadvertent activation of the controller of the anesthesia vaporizer.

2. The system (100) of claim 1, wherein the first filter (10) and the second filter (10) comprise activated charcoal filters to capture anesthetic vapors generated by the anesthesia vaporizer (40).

3. The system (100) of claim 1 or claim 2, wherein the strip (32) comprises a release liner having a first section and a second section, with a label (30) attached to the first section and an adhesive backed-strip attached to the second section of the release liner.

4. The system of claim 3, wherein the adhesive-backed strip (32) further comprises a visual warning that the patient is a malignant hyperthermic patient.

5. The system of any one of claims 3-4, wherein the connector (12) comprises at least one perforated section to attach the adhesive-backed strip (32) to the connector (12) and/or the second section of the release liner.

6. The system of any one of claims 3-5, wherein the release liner includes indicia advising removal of the pair of filters (10) from an anesthesia machine (20) at an end of a procedure performed on the patient.

7. The system of claim 6, wherein the indicia of the release liner becomes visible upon removal of the adhesive-backed strip (32) from the release liner.

8. The system of any one of claims 3-6, wherein the adhesive-backed strip (32) comprises a plurality of adhesive backed strips.

9. The system of claim 8, wherein at least one of the plurality of adhesive-backed strips (32) includes indicia indicating the patient may be susceptible to malignant hyperthermia.

10. The system of any one of claims 1-9, wherein the strip (32) is attached to the connector via a perforated section.

11. The system of claim 1, wherein the strip (32) comprises an adhesive backing for attaching the strip (32) to the connector (12) and for attaching the strip (32) to controls (42) of the anesthesia vaporizer (40).

12. The system of claim 1, wherein the strip (32) comprises a plurality of strips, each strip of the plurality of strips comprising an adhesive backing and including indicia indicating the patient may be susceptible to malignant hyperthermia.

13. The system (100) of any one of claims 3-12, wherein the release liner comprises indicia advising removal of the first filter (10) and second filter (10) from the anesthesia machine (20) at an end of a procedure performed on the patient.

14. The system of any one of claims 2-14, wherein the activated charcoal filters (10) capture an amount of the anesthetic vapors generated by the anesthesia machine ranging from about 30 parts per million (ppm) to 150 ppm, such as 35, 40, 45, 50, 55, 60, 70, 80, 90, 100, 120, 130 ppm or an amount of anesthetic vapors falling within a range defined by any two of the foregoing values.

## Patentansprüche

1. System (100), um zu verhindern, dass Anästhesiedämpfe einen Patienten erreichen, wobei das System ein Aktivkohlefiltersystem aufweist, das einen ersten Filter (10) und einen zweiten Filter (10), einen Verbinder (12), das den ersten Filter (10) mit dem zweiten Filter (10) verbindet, aufweist, wobei sowohl der erste als auch der zweite Filter (10) an einem Anästhesieverdampfer (40) anbringbar ist, **dadurch gekennzeichnet, dass** das System weiterhin aufweist:
einen Streifen (32), der entfernbar an wenigstens einem Abschnitt des Aktivkohlefiltersystems (100) befestigt ist, wobei der Streifen (32) von dem Aktivkohlefiltersystem (100) entfernbar und auf einem Steuergerät (42) des Anästhesieverdampfers (40) platzierbar ist, um eine unbeabsichtigte Aktivierung des Steuergeräts des Anästhesieverdampfers zu verhindern.

2. System (100) nach Anspruch 1, wobei der erste Filter (10) und der zweite Filter (10) Aktivkohlefilter aufweisen, um von dem Anästhesieverdampfer (40) erzeugte Anästhesiedämpfe aufzunehmen.

3. System (100) nach Anspruch 1 oder Anspruch 2, wobei der Streifen (32) einen Abziehstreifen mit einem ersten Abschnitt und einem zweiten Abschnitt aufweist, wobei ein Etikett (30) an dem ersten Abschnitt und ein Klebestreifen an dem zweiten Abschnitt des Abziehstreifens befestigt ist.

4. System nach Anspruch 3, wobei der Klebestreifen (32) weiterhin eine visuelle Warnung aufweist, dass es sich bei dem Patienten um einen Patienten mit maligner Hyperthermie handelt.

5. System nach einem der Ansprüche 3 bis 4, wobei der Verbinder (12) wenigstens einen perforierten Abschnitt aufweist, um den Klebestreifen (32) an dem Verbinder (12) und/oder dem zweiten Abschnitt des Abziehstreifens zu befestigen.

6. System nach einem der Ansprüche 3 bis 5, wobei der Abziehstreifen Hinweise enthält, die empfehlen, das Filterpaar (10) am Ende eines an dem Patienten durchgeführten Eingriffs von einem Anästhesiegerät (20) zu entfernen.

7. System nach Anspruch 6, wobei der Hinweis des Abziehstreifens sichtbar wird, wenn der Klebestreifen (32) von dem Abziehstreifen entfernt wird.

8. System nach einem der Ansprüche 3 bis 6, wobei der Klebestreifen (32) mehrere Klebestreifen aufweist.

9. System nach Anspruch 8, wobei wenigstens einer der Klebestreifen (32) Hinweise enthält, die darauf hinweisen, dass der Patient für maligne Hyperthermie anfällig sein könnte.

10. System nach einem der Ansprüche 1-9, wobei der Streifen (32) über einen perforierten Abschnitt an dem Verbinder befestigt ist.

11. System nach Anspruch 1, wobei der Streifen (32) eine klebende Rückseite aufweist, um den Streifen (32) am Verbinder (12) zu befestigen und um den Streifen (32) an Bedienelementen (42) des Anästhesieverdampfers (40) zu befestigen.

12. System nach Anspruch 1, wobei der Streifen (32) mehrere Streifen aufweist, wobei jeder der Streifen eine klebende Rückseite aufweist und Hinweise enthält, die darauf hinweisen, dass der Patient für maligne Hyperthermie anfällig sein könnte.

13. System (100) nach einem der Ansprüche 3 bis 12, wobei der Abziehstreifen Hinweise aufweist, die empfehlen, den ersten Filter (10) und den zweiten Filter (10) am Ende eines an dem Patienten durchgeführten Eingriffs von dem Anästhesiegerät (20) zu entfernen.

14. System nach einem der Ansprüche 2-14, wobei die Aktivkohlefilter (10) eine Menge an von dem Anästhesiegerät erzeugten Anästhesiedämpfen aufnehmen, die von etwa 30 Teilchen pro Million (ppm) bis 150 ppm reicht, beispielsweise 35, 40, 45, 50, 55, 60, 70, 80, 90, 100, 120, 130 ppm oder eine Menge an Anästhesiedämpfen, die in einen Bereich fällt, der durch zwei der vorgenannten Werte definiert ist.

## Revendications

1. Système (100) pour empêcher que des vapeurs anesthésiques n'atteignent un patient, le système comprenant un système de filtres au charbon actif qui comprend un premier filtre (10) et un second filtre (10), et un connecteur (12) qui connecte le premier filtre (10) au second filtre (10), dans lequel chacun des premier et second filtres (10) est destiné à être lié à un vaporisateur d'anesthésiant (40), **caractérisé en ce que** le système comprend en outre :
une bande (32) liée de façon amovible à au moins une partie du système de filtres au charbon actif (100), la bande (32) étant destinée à être enlevée du système de filtres au charbon actif (100) et à être placée sur un contrôleur (42) du vaporisateur d'anesthésiant (40) pour empêcher l'activation par inadvertance du contrôleur du vaporisateur d'anesthésiant.

2. Système (100) selon la revendication 1, dans lequel le premier filtre (10) et le second filtre (10) comprennent des filtres au charbon actif pour capturer les vapeurs anesthésiques qui sont générées par le vaporisateur d'anesthésiant (40).

3. Système (100) selon la revendication 1 ou la revendication 2, dans lequel la bande (32) comprend une pellicule détachable qui comporte une première section et une seconde section, une étiquette (30) étant liée à la première section et une bande à dos adhésif étant liée à la seconde section de la pellicule détachable.

4. Système selon la revendication 3, dans lequel la bande à dos adhésif (32) comprend en outre un avertissement visuel consistant en ce que le patient est un patient en état d'hyperthermie maligne.

5. Système selon l'une quelconque des revendications 3-4, dans lequel le connecteur (12) comprend au moins une section perforée pour lier la bande à dos adhésif (32) au connecteur (12) et/ou à la seconde section de la pellicule détachable.

6. Système selon l'une quelconque des revendications 3-5, dans lequel la pellicule détachable inclut des inscriptions qui conseillent d'enlever la paire de filtres (10) hors d'un appareil d'anesthésie (20) à la fin d'une procédure réalisée sur le patient.

7. Système selon la revendication 6, dans lequel les inscriptions de la pellicule détachable deviennent visibles suite à l'enlèvement de la bande à dos adhésif (32) par rapport à la pellicule détachable.

8. Système selon l'une quelconque des revendications 3-6, dans lequel la bande à dos adhésif (32) comprend une pluralité de bandes à dos adhésif.

9. Système selon la revendication 8, dans lequel au moins l'une de la pluralité de bandes à dos adhésif (32) inclut des inscriptions qui indiquent que le patient peut être susceptible de développer une hyperthermie maligne.

10. Système selon l'une quelconque des revendications 1-9, dans lequel la bande (32) est liée au connecteur via une section perforée.

11. Système selon la revendication 1, dans lequel la bande (32) comprend un support adhésif pour lier la bande (32) au connecteur (12) et pour lier la bande (32) à des commandes (42) du vaporisateur d'anesthésiant (40).

12. Système selon la revendication 1, dans lequel la bande (32) comprend une pluralité de bandes, chaque bande de la pluralité de bandes comprenant un support adhésif et incluant des inscriptions qui indiquent que le patient peut être susceptible de développer une hyperthermie maligne.

13. Système (100) selon l'une quelconque des revendications 3-12, dans lequel la pellicule détachable comprend des inscriptions qui conseillent d'enlever le premier filtre (10) et le second filtre (10) hors de l'appareil d'anesthésie (20) à la fin d'une procédure réalisée sur le patient.

14. Système selon l'une quelconque des revendications 2-14, dans lequel les filtres au charbon actif (10) capturent une quantité des vapeurs anesthésiques qui sont générées par l'appareil d'anesthésie s'inscrivant dans une plage qui va d'environ 30 parties par million (ppm) à 150 ppm, tel que 35, 40, 45, 50, 55, 60, 70, 80, 90, 100, 120, 130 ppm ou une quantité de vapeurs anesthésiques tombant à l'intérieur d'une plage qui est définie par deux quelconques des valeurs mentionnées ci-avant.
